# EUROPÄISCHE PATENTSCHRIFT

(11) **EP 1 294 946 B1**
(45) Veröffentlichungstag und Bekanntmachung des Hinweises auf die Patenterteilung: **07.04.2004**
(21) Anmeldenummer: 01953183.9
(22) Anmeldetag: 29.06.2001
(51) Int. Cl.: C12Q 1/68, B01J 19/00

(54) **MULTIPLEX-SEQUENZIERUNGSVERFAHREN**
MULTIPLEX SEQUENCING METHOD
PROCEDE DE SEQUENçAGE MULTIPLEX

(30) Priorität: 30.06.2000 DE 10031842
(43) Veröffentlichungstag der Anmeldung: 26.03.2003
(73) Patentinhaber: Gnothis Holding SA, 1015 Ecublens (CH)
(72) Erfinder: RIGLER, Rudolf, CH-1025 St-Sulpice (CH)
(74) Vertreter: Weiss, Wolfgang, Dipl.-Chem. Dr.
(86) Internationale Anmeldenummer: PCT/EP2001/007462
(87) Internationale Veröffentlichungsnummer: WO 2002/002795

(56) Entgegenhaltungen:
- WO-A-99/13109
- DE-A- 10 065 626
- US-A- 4 849 077
- US-A- 4 962 037
- STEPHAN J ET AL: "Towards a general procedure for sequencing single DNA molecules" JOURNAL OF BIOTECHNOLOGY, ELSEVIER SCIENCE PUBLISHERS, AMSTERDAM, NL, Bd. 86, Nr. 3, 13. April 2001 (2001-04-13), Seiten 255-267, XP004230519 ISSN: 0168-1656
- FOELDES-PAPP ZENO ET AL: "Fluorescent high-density labeling of DNA: Error-free substitution for a normal nucleotide." JOURNAL OF BIOTECHNOLOGY, Bd. 86, Nr. 3, 2001, Seiten 237-253, XP004230518 ISSN: 0168-1656
- FOELDES-PAPP ZENO ET AL: "Fluorescently labeled model DNA sequences for exonucleolytic sequencing." JOURNAL OF BIOTECHNOLOGY, Bd. 86, Nr. 3, 2001, Seiten 203-224, XP004230516 ISSN: 0168-1656
- SAUER M ET AL: "Single molecule DNA sequencing in submicrometer channels: State of the art and future prospects." JOURNAL OF BIOTECHNOLOGY, Bd. 86, Nr. 3, 2001, Seiten 181-201, XP004230515 ISSN: 0168-1656

## Beschreibung

Die Erfindung betrifft ein Verfahren und eine Vorrichtung zur Multiplex-Sequenzierung von auf einem Träger immobilisierten Nukleinsäuremolekülen.

Die Sequenzierung des aus ca. 3 x 10⁹ Basen bestehenden humanen Genoms oder des Genoms anderer Organismen sowie die Bestimmung und der Vergleich individueller Sequenzvarianten erfordert die Bereitstellung von Sequenziermethoden, die einerseits schnell sind und andererseits routinemäßig und mit geringen Kosten eingesetzt werden können. Obwohl große Anstrengungen unternommen worden sind, um gängige Sequenziermethoden, z.B. die enzymatische Kettenabbruchmethode nach Sanger et al. (Proc. Natl. Acad. Sci. USA 74 (1977) 5463), zu beschleunigen, insbesondere durch Automatisierung (Adams et al., Automated DNA Sequencing and Analysis (1994), New York, Academic Press) können derzeit maximal nur 2.000 Basen pro Tag mit einem Sequenziergerät bestimmt werden.

Während der letzten Jahre sind neue Ansätze zur Überwindung der Beschränkungen konventioneller Sequenzierverfahren entwickelt worden, u.a. die Sequenzierung durch Rastertunnelmikroskopie (Lindsay und Phillip, Gen. Anal. Tech. Appl. 8 (1991), 8-13), durch hochparallelisierte Kapillarelektrophorese (Huang et al., Anal. Chem. 64 (1992), 2149-2154; Kambara und Takahashi, Nature 361 (1993), 565-566), durch Oligonukleotidhybridisierung (Drmanac et al., Genomics 4 (1989), 114-128; Khrapko et al., FEBS Let. 256 (1989), 118-122; Maskos und Southern, Nucleic Acids Res. 20 (1992), 1675-1678 und 1679-1684) sowie durch Matrix-unterstützte Laser-Desorptions/Ionisierungs-Massenspektroskopie (Hillenkamp et al., Anal. Chem. 63 (1991), 1193A-1203A).

Ein weiterer Ansatz ist die Einzelmolekülsequenzierung (Dörre et al., Bioimaging 5 (1997), 139-152), bei der die Sequenz von Nukleinsäuren durch fortschreitenden enzymatischen Abbau von fluoreszenzmarkierten einzelsträngigen DNA-Molekülen und Nachweis der sequenziell freigesetzten Monomermoleküle in einem Mikrostrukturkanal erfolgt. Der Vorteil dieses Verfahrens besteht darin, dass nur ein einziges Mokeül der Zielnukleinsäure für die Durchführung einer Sequenzbestimmung ausreicht.

Obwohl durch Anwendung der oben genannten Methoden bereits erhebliche Fortschritte erzielt wurden, besteht ein großes Bedürfnis nach weiteren Verbesserungen. Die der vorliegenden Erfindung zugrundeliegende Aufgabe bestand somit darin, ein Verfahren zur Sequenzierung von Nukleinsäuren bereitzustellen, das eine weitere Verbesserung gegenüber dem Stand der Technik darstellt und das eine parallele Bestimmung einzelner Nukleinsäuremoleküle in einem Multiplexformat ermöglicht.

Diese Aufgabe wird gelöst durch ein Verfahren zur Sequenzierung von Nukleinsäuren, umfassend die Schritte:
(a) Bereitstellen eines Trägers mit einer Vielzahl von darauf immobilisierten Nukleinsäuremolekülen, wobei die Nukleinsäuremoleküle mehrere Fluoreszenzmarkierungsgruppen tragen,
(b) fortschreitendes Abspalten einzelner Nukleotidbausteine von den immobilisierten Nukleinsäuremolekülen und
(c) gleichzeitiges Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine.

Das erfindungsgemäße Verfahren ist eine trägergestützte Multiplex-Sequenziermethode, bei der eine Vielzahl von immobilisierten Nukleinsäuremolekülen gleichzeitig untersucht wird. Der für das Verfahren verwendete Träger kann ein beliebiger planarer oder strukturierter Träger sein, der zur Immobilisierung von Nukleinsäuremolekülen geeignet ist. Beispiele für geeignete Trägermaterialien sind Glas, Kunststoff, Metalle oder Halbmetalle wie etwa Silicium, Metalloxide wie Siliciumoxid etc. Auch die Gestaltung des Trägers kann grundsätzlich beliebig sein, sofern ein Reaktionsraum gebildet werden kann, welcher das fortschreitende Abspalten einzelner Nukleotidbausteine von den auf dem Träger immobilisierten Nukleinsäuren in einem flüssigen Reaktionsgemisch ermöglicht.

Die Nukleinsäuremoleküle.werden vorzugsweise über ihre 5'- oder 3'-Enden auf dem Träger immobilisiert, wobei sie in einzelsträngiger Form oder in doppelsträngiger Form vorliegen können. Bei doppelsträngigen Molekülen muss sichergestellt sein, dass markierte Nukleotidbausteine nur von einem einzigen Strang abgespalten werden können. Die Bindung der Nukleinsäuremoleküle an den Träger kann durch kovalente oder nicht kovalente Wechselwirkungen erfolgen. Beispielsweise kann die Bindung der Polynukleotide an den Träger durch hochaffine Wechselwirkungen zwischen den Partnern eines spezifischen Bindepaares, z.B. Biotin/Streptavidin oder Avidin, Hapten/Anti-Hapten-Antikörper, Zucker/Lectin etc., vermittelt werden. So können biotinylierte Nukleinsäuremoleküle an Streptavidinbeschichtete Träger gekoppelt werden. Alternativ können die Nukleinsäuremoleküle auch adsorptiv an den Träger gebunden werden. So kann eine Bindung von durch Einbau von Alkanthiolgruppen modifizierten Nukleinsäuremolekülen an metallische Träger, z.B. Goldträger, erfolgen. Noch eine weitere Alternative ist die kovalente Immobilisierung, wobei die Bindung der Polynukleotide über reaktive Silangruppen auf einer Silika-Oberfläche vermittelt werden kann.

An einen Träger werden mehrere, für eine Sequenzierung vorgesehene Nukleinsäuremoleküle gebunden. Vorzugsweise werden mindestens 100, besonders bevorzugt mindestens 1.000 und besonders bevorzugt mindestens 10.000 und bis zu mehr als 10⁶ Nukleinsäuremoleküle an den Träger gebunden. Die gebundenen Nukleinsäurefragmente haben eine Länge von vorzugsweise 200 bis 2.000 Nukleotide, besonders bevorzugt 400 bis 1.000 Nukleotide. Die an den Träger gebundenen Nukleinsäuremoleküle, z.B. DNA-Moleküle oder RNA-Moleküle, enthalten mehrere Fluoreszenzmarkierungsgruppen, wobei vorzugsweise mindestens 50 %, besonders bevorzugt mindestens 70 % und am meisten bevorzugt im wesentlichen alle, z.B. mindestens 90 %, der Nukleotidbausteine von einem Basentyp eine Fluoreszenzmarkierungsgruppe tragen. Derart markierte Nukleinsäuren können durch enzymatische Primerextension an einer Nukleinsäurematrize unter Verwendung einer geeigneten Polymerase, z.B. einer DNA-Polymerase wie etwa Taq-Polymerase, einer thermostabilen DNA-Polymerase von Thermococcus gorgonarius oder anderen thermostabilen Organismen (Hopfner et al., PNAS USA 96 (1999), 3600-3605) oder einer mutierten Taq-Polymerase (Patel und Loeb, PNAS USA 97 (2000), 5095-5100) unter Verwendung fluoreszenzmarkierter Nukleotidbausteine erzeugt werden.

Die markierten Nukleinsäuremoleküle können auch durch Amplifikationsreaktionen, z.B. PCR, hergestellt werden. So entstehen bei einer asymmetrischen PCR Amplifikationsprodukte, bei denen nur einziger Strang Fluroeszenzmarkierungen enthält. Derartige asymmetrische Amplifikationsprodukte können in doppelsträngiger Form sequenziert werden. Durch symmetrische PCR werden Nukleinsäurefragmente hergestellt, bei denen beide Stränge fluoreszenzmarkiert sind. Diese beiden fluoreszenzmarkierten Stränge können separiert und getrennt in einzelsträngiger Form immobilisiert werden, so daß die Sequenz eines oder beider Komplementärstränge separat bestimmt werden kann. Alternativ kann einer der beiden Stränge am 3'-Ende derart modifiziert werden, z.B. durch Einbau einer PNA-Klammer, sodass eine Abspaltung von Monomerbausteinen nicht mehr möglich ist. In diesem Fall ist eine Doppelstrangsequenzierung möglich.

Vorzugsweise tragen im Wesentlichen alle Nukleotidbausteine von mindestens zwei Basentypen, beispielsweise zwei, drei oder vier Basentypen, eine Fluoreszenzmarkierung, wobei jeder Basentyp günstigerweise eine unterschiedliche Fluoreszenzmarkierungsgruppe trägt. Wenn die Nukleinsäuremoleküle nicht vollständig markiert sind, so kann durch parallele Sequenzierung von mehreren Molekülen dennoch die Sequenz vollständig bestimmt werden.

Die Nukleinsäurematrize, deren Sequenz bestimmt werden soll, kann beispielsweise aus DNA-Matrizen wie genomischen DNA-Fragmenten, cDNA-Molekülen, Plasmiden etc., aber auch aus RNA-Matrizen wie mRNA-Mokeülen ausgewählt werden.

Die Fluoreszenzmarkierungsgruppen können aus bekannten zur Markierung von Biopolymeren, z.B. Nukleinsäuren, verwendeten Fluoreszenzmarkierungsgruppen, wie etwa Fluorescein, Rhodamin, Phycoerythrin, Cy3, Cy5 oder Derivaten davon etc. ausgewählt werden.

Das erfindungsgemäße Verfahren beruht darauf, dass in Nukleinsäurestränge eingebaute Fluoreszenzmarkierungsgruppen Wechselwirkungen mit benachbarten Gruppen, beispielsweise mit chemischen Gruppen der Nukleinsäuren, insbesondere Nukleobasen wie etwa G, oder/und benachbarten Fluoreszenzmarkierungsgruppen eingehen, die zu einer Änderung der Fluoreszenz, insbesondere der Fluoreszenzintensität gegenüber den Fluoreszenzmarkierungsgruppen in "isolierter" Form aufgrund von Quench- oder/und Energietransfer-Vorgängen führen. Durch das Abspalten einzelner Nukleotidbausteine verändert sich die Gesamtfluoreszenz, z.B. die Fluoreszenzintensität eines immobilisierten Nukleinsäurestranges abhängig von der Abspaltung einzelner Nukleotidbausteine, d.h. abhängig von der Zeit. Diese zeitliche Änderung der Fluoreszenz kann parallel für eine Vielzahl von Nukleinsäuremolekülen erfasst und mit der Basenfolge der einzelnen Nukleinsäurestränge korreliert werden. Vorzugsweise werden solche Fluoreszenzmarkierungsgruppen verwendet, die, wenn sie in den Nukleinsäurestrang eingebaut sind, zumindest teilweise gequencht sind, so dass nach Abspaltung des die Markierungsgruppe enthaltenden Nukleotidbausteins oder eines benachbarten Bausteins, der ein Quenchen verursacht, die Fluoreszenzintensität erhöht wird.

Die Sequenzierungsreaktion des erfindungsgemäßen Verfahrens umfasst das fortschreitende Abspalten einzelner Nukleotidbausteine von den immobilisierten Nukleinsäuremolekülen. Vorzugsweise erfolgt eine enzymatische Abspaltung unter Verwendung einer Exonuklease, wobei Einzelstrang- bzw. Doppelstrang-Exonukleasen, die in 5'→3'-Richtung oder 3'→5'-Richtung abbauen - je nach Art der Immobilisierung der Nukleinsäurestränge auf dem Träger - eingesetzt werden können. Besonders bevorzugt werden als Exonukleasen T7 DNA-Polymerase, E.coli Exonuklease I oder E.coli Exonuklease III verwendet.

Während der fortschreitenden Abspaltung einzelner Nukleotidbausteine kann eine Änderung der Fluoreszenzintensität des immobilisierten Nukleinsäurestrangs oder/und des abgespaltenen Nukleotidbausteins aufgrund von Quench- oder Energietransfervorgängen gemessen werden. Diese zeitliche Änderung der Fluoreszenzintensität ist von der Basenfolge des untersuchten Nukleinsäurestrangs abhängig und kann daher mit der Sequenz korreliert werden. Zur vollständigen Sequenzbestimmung eines Nukleinsäurestrangs werden üblicherweise mehrere - an unterschiedlichen Basen, z.B. A, G, C und T bzw. Kombinationen von zwei verschiedenen Basen - markierte Nukleinsäurestränge vorzugsweise durch enzymatische Primerextension, wie zuvor beschrieben, erzeugt und auf dem Träger immobilisiert, wobei die Immobilisierung an statistischen Orten des Trägers oder aber auch ortsspezifisch erfolgen kann. Gegebenenfalls kann an den zu untersuchenden Nukleinsäurestrang noch ein "Sequenzidentifikator", d.h. eine markierte Nukleinsäure bekannter Sequenz, angefügt werden, z.B. durch enzymatische Reaktion mit Ligase oder/und Terminaler Transferase, sodass zu Beginn der Sequenzierung zunächst ein bekanntes Fluoreszenzmuster und anschließend erst das der unbekannten, zu untersuchenden Sequenz entsprechende Fluoreszenzmuster erhalten wird. Insgesamt werden auf einem Träger vorzugsweise 10³ bis 10⁶ Nukleinsäurestränge immobilisiert.

Um die Entfernung abgespaltener Nukleotidbausteine von den immobilisierten Nukleotidsträngen zu beschleunigen, wird im Reaktionsraum vorzugsweise ein Konvektionsfluss vom Träger weg erzeugt. Die Flussgeschwindigkeit kann dabei im Bereich von 1 bis 10 mm/s liegen.

Die Detektion umfasst vorzugsweise eine Mehrpunkt-Fluoreszenzanregung durch Laser, z.B. eine Punktmatrix von Laserpunkten erzeugt durch eine Diffraktionsoptik oder einen Quanten-Well-Laser. Die durch Anregung erzeugte Fluoreszenzemission mehrerer Nukleinsäurestränge kann durch eine Detektormatrix erzeugt werden, die beispielsweise eine elektronische Detektormatrix, z.B. eine CCD-Kamera oder eine Avalanche-Fotodiodenmatrix, umfasst. Die Detektion kann derart erfolgen, dass Fluoreszenzanregung und Detektion an allen untersuchten Nukleinsäuresträngen parallel erfolgt. Alternativ dazu kann in mehreren Schritten jeweils ein Teil der Nukleinsäurestränge unter Verwendung einer Submatrix von Laserpunkten und Detektoren vorzugsweise unter Verwendung einer Hochgeschwindigkeitsscannerprozedur untersucht werden.

Ein weiterer Gegenstand der Erfindung ist ein Träger zur Sequenzierung von Nukleinsäuren, umfassend eine Vielzahl von darauf immobilisierten Nukleinsäuremolekülen, wobei die Nukleinsäuremoleküle in einzelsträngiger Form vorliegen und mehrere Fluoreszenzmarkierungsgruppen tragen.

Noch ein weiterer Gegenstand der Erfindung ist eine Vorrichtung zur Sequenzierung von Nukleinsäuren, umfassend
(a) einen Träger wie zuvor spezifiziert,
(b) einen Reaktionsraum zum fortschreitenden Abspalten einzelner Nukleotidbausteine von den immobilisierten Nukleinsäuremolekülen und
(c) Mittel zum gleichzeitigen Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine.

Das erfindungsgemäße Verfahren kann beispielsweise für die Analyse von Genomen und Transkriptomen bzw. für differenzielle Analysen, z.B. Untersuchungen bezüglich des Unterschieds im Genom bzw. Transkriptom einzelner Spezies oder Organismen innerhalb einer Spezies eingesetzt werden.

Weiterhin soll die vorliegende Erfindung durch die nachfolgenden Figuren erläutert werden. Es zeigen:
Figur 1:
   (A) die schematische Darstellung eines erfindungsgemäßen Trägers (2) mit einer Vielzahl von daran immobilisierten einzelsträngigen Nukleinsäuremolekülen (4). Ein Träger mit einer Fläche von 1 bis 2 cm² kann beispielsweise bis zu 10⁶ Nukleinsäurestränge enthalten.
   (B) die an den Träger (2) immobilisierten Nukleinsäuremoleküle (4) können einen 5'-biotinylierten (oder mit einer anderen Festphasenbindegruppe versehenen) Primer (4a), einen zu sequenzierenden fluoreszenzmarkierten Abschnitt (4b) und gegebenenfalls einen fluoreszenzmarkierten Sequenzidentifikator (4c) umfassen. Durch Einwirkung einer Exonuklease (6) werden fortlaufend einzelne Nukleotidbausteine (8) abgespalten. Während die in den Nukleinsäurestrang eingebauten Nukleotidbausteine aufgrund von Quenchvorgängen keine oder nur eine geringe Fluoreszenz zeigen, wird die Fluoreszenz nach der Abspaltung erhöht.
Figur 2:
   (A) eine erste Ausführungsform der Erfindung, wobei auf die auf dem Träger (2) immobilisierten Nukleinsäuremoleküle (4) von einem Laser (6) mit Anregungslicht (8) bestrahlt werden, welches von einem diffraktionsoptischen Element (10) auf die einzelnen immobilisierten Nukleinsäuresträngegelenktwird. Das Fluoreszenzemissionslicht(12) wird von einer Detektormatrix (14), z.B. einer CCD-Kamera, aufgezeichnet.
   (B) In einer weiteren Ausführungsform der Erfindung wird ein Träger (20) mit darin integriertem Quanten-Well-Laser (60) eingesetzt.

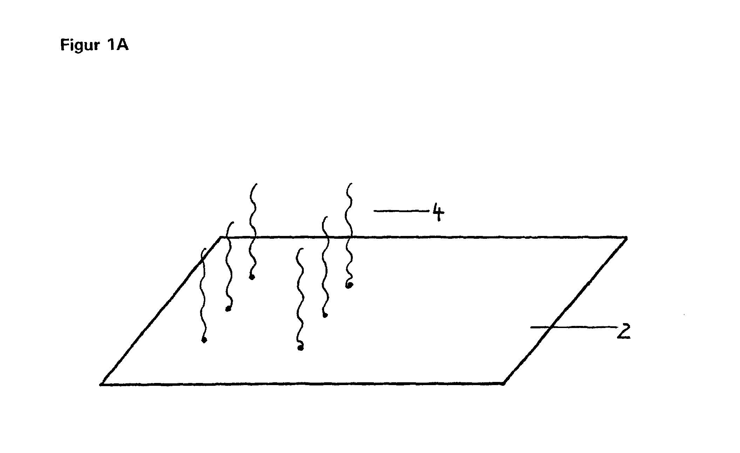

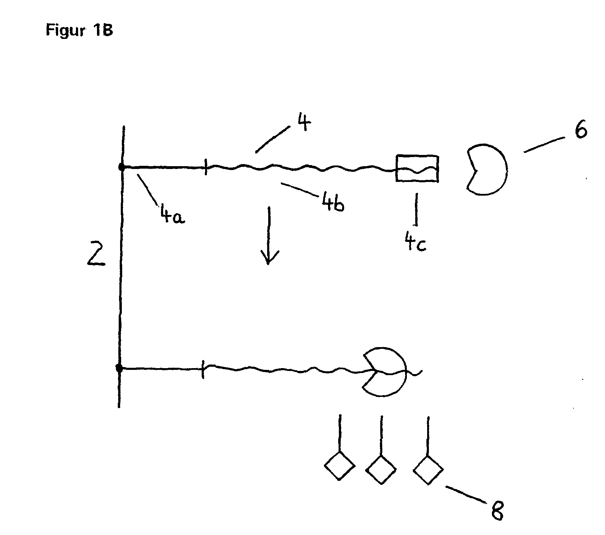

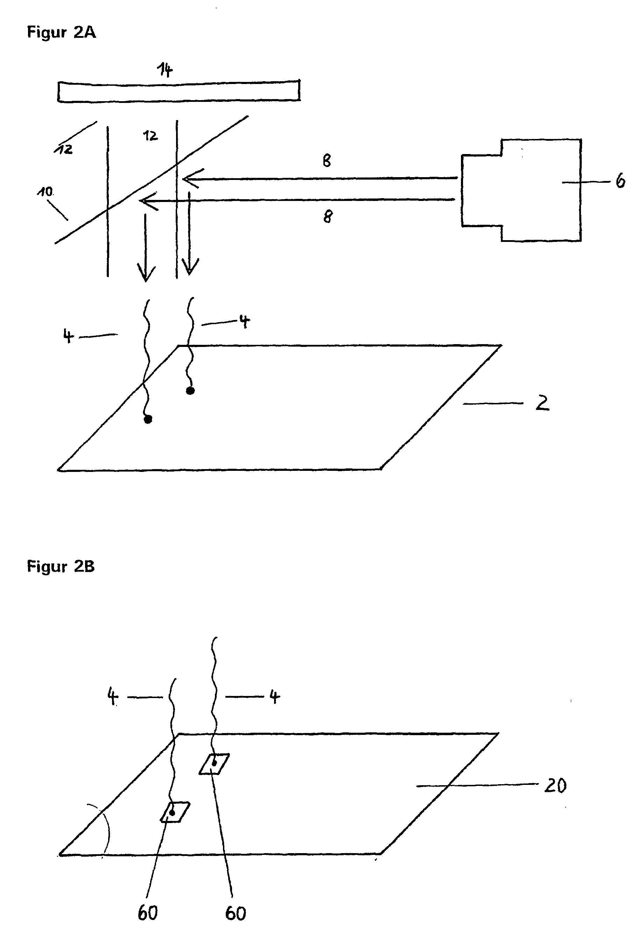

## Patentansprüche

1. Verfahren zur Sequenzierung von Nukleinsäuren, umfassend die Schritte:
(a) Bereitstellen eines Trägers mit einer Vielzahl von darauf immobilisierten Nukleinsäuremolekülen, wobei die Nukleinsäuremoleküle mehrere Fluoreszenzmarkierungsgruppen tragen,
(b) fortschreitendes Abspalten einzelner Nukleotidbausteine von den immobilisierten Nukleinsäuremolekülen und
(c) gleichzeitiges Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine.

2. Verfahren nach Anspruch 1,
**dadurch gekennzeichnet,**
**dass** man einen planaren Träger verwendet.

3. Verfahren nach Anspruch 1,
**dadurch gekennzeichnet,**
**dass** man einen strukturierten Träger verwendet.

4. Verfahren nach einem der Ansprüche 1 bis 3,
**dadurch gekennzeichnet,**
**dass** die Nukleinsäuremoleküle über ihre 5'- oder 3'-Enden auf dem Träger immobilisiert werden.

5. Verfahren nach einem der Ansprüche 1 bis 4,
**dadurch gekennzeichnet,**
**dass** die Nukleinsäuremoleküle in einzelsträngiger Form auf dem Träger immobilisiert werden.

6. Verfahren nach einem der Ansprüche 1 bis 4,
**dadurch gekennzeichnet,**
**dass** die Nukleinsäuremoleküle in doppelsträngiger Form auf dem Träger immobilisiert werden, wobei markierte Nukleotidbausteine nur von einem einzigen Strang abgespalten werden können.

7. Verfahren nach einem der Ansprüche 1 bis 6,
**dadurch gekennzeichnet,**
**dass** die Nukleinsäuremoleküle derart markiert sind, dass mindestens 50 % aller Nukleotidbausteine von einem Basentyp eine Fluoreszenzmarkierungsgruppe tragen.

8. Verfahren nach Anspruch 7,
**dadurch gekennzeichnet,**
**dass** im Wesentlichen alle Nukleotidbausteine von einem Basentyp eine Fluoreszenzmarkierungsgruppe tragen.

9. Verfahren nach einem der Ansprüche 1 bis 8,
**dadurch gekennzeichnet,**
**dass** das Abspalten einzelner Nukleotidbausteine durch eine Exonuklease erfolgt.

10. Verfahren nach Anspruch 9,
**dadurch gekennzeichnet,**
**dass** T7 DNA-Polymerase, E.coli Exonuklease I oder E.coli Exonuklease III verwendet wird.

11. Verfahren nach einem der vorhergehenden Ansprüche,
**dadurch gekennzeichnet,**
**dass** das Bestimmen der Basenfolge eine Mehrpunkt-Fluoreszenzanregung durch Laser umfasst.

12. Verfahren nach einem der vorhergehenden Ansprüche,
**dadurch gekennzeichnet,**
**dass** das Bestimmen der Basenfolge eine Detektion der Fluoreszenzemission mehrerer Nukleinsäurestränge durch eine Detektionsmatrix umfasst.

13. Verfahren nach Anspruch 12,
**dadurch gekennzeichnet,**
**dass** eine CCD-Kamera oder eine Avalanche-Fotodiodenmatrix verwendet wird.

14. Verfahren nach einem der vorhergehenden Ansprüche,
**dadurch gekennzeichnet,**
**dass** Fluoreszenzanregung und -detektion an allen untersuchten Nukleinsäuresträngen parallel erfolgt.

15. Verfahren nach einem der Ansprüche 1 bis 13,
**dadurch gekennzeichnet,**
**dass** Fluoreszenzanregung und -detektion in mehreren Schritten jeweils an einen Teil der untersuchten Nukleinsäurestränge unter Verwendung einer Submatrix von Laserpunkten und Detektoren erfolgt.

16. Verfahren nach einem der vorhergehenden Ansprüche,
**dadurch gekennzeichnet,**
**dass** während der Bestimmung ein Konvektionsfluss vom Träger weg erzeugt wird.

17. Verfahren nach einem der vorhergehenden Ansprüche,
**dadurch gekennzeichnet,**
**dass** die Fluoreszenzmarkierungsgruppen, wenn sie in die Nukleinsäurestränge eingebaut sind, zumindest teilweise gequencht sind, und dass nach Abspaltung die Fluoreszenzintensität erhöht wird.

18. Träger zur Sequenzierung von Nukleinsäuren, umfassend eine Vielzahl von darauf immobilisierten Nukleinsäuremolekülen, wobei die Nukleinsäuremoleküle in einzelsträngiger Form vorliegen und mehrere Fluoreszenzmarkierungsgruppen tragen.

19. Träger nach Anspruch 18,
**dadurch gekennzeichnet,**
**dass** die Nukleinsäuremoleküle derart markiert sind, dass mindestens 50 % aller Nukleotidbausteine von einem Basentyp eine Fluoreszenzmarkierungsgruppe tragen.

20. Träger nach Anspruch 18 oder 19,
**dadurch gekennzeichnet,**
**dass** die Nukleinsäuremoleküle eine Länge von 200 bis 2000 Nukleotide aufweisen.

21. Vorrichtung zur Sequenzierung von Nukleinsäuren, umfassend
(a) einen Träger nach einem der Ansprüche 18 bis 20,
(b) einen Reaktionsraum zum fortschreitenden Abspalten einzelner Nukleotidbausteine von den immobilisierten Nukleinsäuremolekülen und
(c) Mittel zum gleichzeitigen Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine.

22. Verwendung des Trägers nach einem der Ansprüche 18 bis 20 oder der Vorrichtung nach Anspruch 21 in einem Verfahren nach einem der Ansprüche 1 bis 17.

## Claims

1. Method for sequencing nucleic acids, which comprises the following steps:
(a) providing a support with a multiplicity of nucleic acid molecules immobilized thereon, said nucleic acid molecules carrying a plurality of fluorescent labelling groups,
(b) progressively removing by cleavage individual nucleotide building blocks from said immobilized nucleic acid molecules and
(c) determining simultaneously the base sequence of a plurality of nucleic acid molecules owing to the time-dependent change in fluorescence of said nucleic acid molecules or/and the nucleotide building blocks removed by cleavage, which change is caused by removing said nucleotide building blocks by cleavage.

2. Method according to Claim 1, **characterized in that** a planar support is used.

3. Method according to Claim 1, **characterized in that** a structured support is used.

4. Method according to any of Claims 1 to 3, **characterized in that** the nucleic acid molecules are immobilized on the support via their 5' or 3' ends.

5. Method according to any of Claims 1 to 4, **characterized in that** the nucleic acid molecules are immobilized on the support in single-stranded form.

6. Method according to any of Claims 1 to 4, **characterized in that** the nucleic acid molecules are immobilized on the support in double-stranded form, it being possible to remove labelled nucleotide building blocks by cleavage only from one single strand.

7. Method according to any of Claims 1 to 6, **characterized in that** the nucleic acid molecules are labelled so that at least 50% of all nucleotide building blocks of a single base type carry a fluorescent labelling group.

8. Method according to Claim 7, **characterized in that** essentially all nucleotide building blocks of a single base type carry a fluorescent labelling group.

9. Method according to any of Claims 1 to 8, **characterized in that** individual nucleotide building blocks are removed by cleavage by an exonuclease.

10. Method according to Claim 9, **characterized in that** T7 DNA polymerase, E. coli exonuclease I or E. coli exonuclese III is used.

11. Method according to any of the preceding claims, **characterized in that** determining the base sequence comprises a multipoint fluorescence excitation by lasers.

12. Method according to any of the preceding claims, **characterized in that** determining the base sequence comprises detection of the fluorescence emission of a plurality of nucleic acid strands via a detection matrix.

13. Method according to Claim 12, **characterized in that** a CCD camera or an avalanche photodiode matrix is used.

14. Method according to any of the preceding claims, **characterized in that** fluorescence excitation and fluorescence detection are carried out in parallel on all nucleic acid strands studied.

15. Method according to any of claims 1 to 13, **characterized in that** fluorescence excitation and fluorescence detection are carried out in several steps, in each case on a portion of the nucleic acid strands studied, using a submatrix of laser dots and detectors.

16. Method according to any of the preceding claims, **characterized in that** a convectional flow away from the support is generated during determination.

17. Method according to any of the preceding claims, **characterized in that** the fluorescent labelling groups are, at least partially, quenched when incorporated into the nucleic acid strands and that the fluorescence intensity is increased after removal by cleavage.

18. Support for sequencing nucleic acids, which comprises a multiplicity of nucleic acid molecules immobilized thereon, said nucleic acid molecules being in single-stranded form and carrying a plurality of fluorescent labelling groups.

19. Support according to Claim 18, **characterized in that** the nucleic acid molecules are labelled so that at least 50% of all nucleotide building blocks of a single base type carry a fluorescent labelling group.

20. Support according to either of Claims 18 and 19, **characterized in that** the nucleic acid molecules are from 200 to 2 000 nucleotides in length.

21. Device for sequencing nucleic acids, comprising
(a) a support according to any of Claims 18 to 20
(b) a reaction space for progressively removing by cleavage individual nucleotide building blocks from said immobilized nucleic acid molecules and
(c) means for determining simultaneously the base sequence of a plurality of nucleic acid molecules owing to the time-dependent change in fluorescence of said nucleic acid molecules or/and the nucleotide building blocks removed by cleavage, which change is caused by removing said nucleotide building blocks by cleavage.

22. Use of the support according to any of Claims 18 to 20 or of the device according to Claim 21 in a method according to any of Claims 1 to 17.

## Revendications

1. Procédé de séquençage d'acides nucléiques, comprenant les étapes de :
(a) mettre à disposition un support ayant une multitude de molécules d'acides nucléiques immobilisées sur celui-ci, les molécules d'acides nucléiques portant plusieurs groupes marqueurs de fluorescence,
(b) séparer progressivement les différents composants nucléotides des molécules d'acides nucléiques immobilisées et
(c) déterminer simultanément la suite de bases de plusieurs molécules d'acides nucléiques sur la base de la modification de fluorescence des molécules d'acides nucléiques ou/et des composants nucléotides séparés, dépendante du temps, provoquée lors de la séparation des composants nucléotides.

2. Procédé selon la revendication 1, **caractérisé en ce que** l'on utilise un support plane.

3. Procédé selon la revendication 1, **caractérisé en ce que** l'on utilise un support structuré.

4. Procédé selon l'une quelconque des revendications 1 à 3, **caractérisé en ce que** les molécules d'acides nucléiques sont immobilisées sur le support par le biais de leurs extrémités 5' et 3'.

5. Procédé selon l'une quelconque des revendications 1 à 4, **caractérisé en ce que** les molécules d'acides nucléiques sous forme de brin simple sont immobilisées sur le support.

6. Procédé selon l'une quelconque des revendications 1 à 4, **caractérisé en ce que** les molécules d'acide nucléique sous forme brin double sont immobilisées sur le support, les composants nucléotides marqués ne pouvant être séparés que d'un seul brin.

7. Procédé selon l'une quelconque des revendications 1 à 6, **caractérisé en ce que** les molécules d'acides nucléiques sont marquées de telle façon qu'au moins 50 % de tous les composants nucléotides d'un type de base portent un groupe marqueur de fluorescence.

8. Procédé selon la revendication 7, **caractérisé en ce que** sensiblement tous les composants nucléotides d'un type de base portent un groupe marqueur de fluorescence.

9. Procédé selon l'une quelconque des revendications 1 à 8, **caractérisé en ce que** la séparation des différents composants nucléotides s'effectue par le biais d'une exonucléase.

10. Procédé selon la revendication 9, **caractérisé en ce que** l'on utilise l'ADN-polymérase T7, l'exonucléase I de E. coli ou l'exonucléase III de E. coli.

11. Procédé selon l'une quelconque des revendications précédentes, **caractérisé en ce que** la détermination de la suite de bases comprend une excitation de fluorescence multiples en points (Mehrpunkt-Fluoreszenzanregung) au laser.

12. Procédé selon l'une quelconque des revendications précédentes, **caractérisé en ce que** la détermination de la suite de bases comprend une détection de l'émission de fluorescence de plusieurs brins d'acides nucléiques par le biais d'une matrice de détection.

13. Procédé selon la revendication 12, **caractérisé en ce que** l'on utilise une caméra CCD ou une matrice de photodiodes photographiques.

14. Procédé selon l'une quelconque des revendications précédentes, **caractérisé en ce que** l'excitation de fluorescence et la détection de fluorescence s'effectuent parallèlement sur tous les brins d'acides nucléiques analysés.

15. Procédé selon l'une quelconque des revendications 1 à 13, **caractérisé en ce que** l'excitation de fluorescence et la détection de fluorescence s'effectuent en plusieurs étapes à chaque fois sur une partie des brins d'acides nucléiques analysés en utilisant une sous-matrice de points laser et de détecteurs.

16. Procédé selon l'une quelconque des revendications précédentes, **caractérisé en ce que**, pendant la détermination, un flux de convection partant du support est produit.

17. Procédé selon l'une quelconque des revendications, **caractérisé en ce que** les groupes marqueurs de fluorescence, quand ils sont intégrés aux brins d'acide nucléique, sont du moins partiellement éteints et que, après la séparation, l'intensité de fluorescence augmente.

18. Supports pour le séquençage des acides nucléiques, comprenant une multitude de molécules d'acides nucléiques immobilisées sur celui-ci, les molécules d'acides nucléiques se présentant sous forme brin simple et portant plusieurs groupes marqueurs de fluorescence.

19. Supports selon la revendication 18, **caractérisés en ce que** les molécules d'acides nucléiques sont marquées de telle sorte que 50 % de tous les composants nucléotides d'un type de base portent un groupe marqueur de fluorescence.

20. Supports selon la revendication 18 ou 19, **caractérisés en ce que** les molécules d'acides nucléiques présentent une longueur d'onde de 200 à 2000 nucléotides.

21. Dispositif de séquençage d'acides nucléiques, comprenant
(a) un support selon l'une des revendications 18 à 20,
(b) une chambre de réaction pour la séparation progressive des différents composants nucléotides des molécules d'acides nucléiques immobilisées et
(c) des moyens de détermination simultanée de la suite de bases de plusieurs molécules d'acides nucléiques sur la base de la modification de fluorescence des molécules d'acides nucléiques ou/et des composants nucléotides séparés, dépendante du temps.

22. Utilisation du support selon l'une quelconque des revendications 18 à 20 ou du dispositif selon la revendication 21 dans un procédé selon l'une quelconque des revendications 1 à 17.
